**Europäisches Patentamt**

**European Patent Office**

**Office européen des brevets**

(11) Numéro de publication: **0 352 207**
**A1**

# (12) DEMANDE DE BREVET EUROPEEN

(21) Numéro de dépôt: **89440076.1**

(22) Date de dépôt: **17.07.89**

(51) Int. Cl.⁵: **A 61 F 13/15**

(30) Priorité: **18.07.88 FR 8810029**
**18.07.88 FR 8810030**

(43) Date de publication de la demande:
**24.01.90 Bulletin 90/04**

(84) Etats contractants désignés:
**AT BE CH DE ES FR GB GR IT LI LU NL SE**

(71) Demandeur: **CELATOSE Société anonyme dite:**
**47, Rue de Bradford**
**F-59200 Tourcoing (Nord) (FR)**

(72) Inventeur: **Bruneau, Jean-Pierre**
**30 rue de la Visterie**
**Nomain F-59310 Orchies (Nord) (FR)**

(74) Mandataire: **Lepage, Jean-Pierre**
**Cabinet Lepage & Aubertin Innovations et Prestations**
**23/25, rue Nicolas Leblanc B.P. 1069**
**F-59011 Lille Cédex 1 (Nord) (FR)**

(54) **Couche-culotte.**

(57) L'invention est relative à une couche culotte qui trouvera notamment son application dans le domaine de l'hygiène infantile et/ou médicale.

La couche (1) est formée d'une feuille extérieure imperméable (5), d'une feuille intérieure perméable (6), d'un matelas absorbant (7), de moyens élastiques longitudinaux (13, 14) d'entrejambe, et de moyens (15, 16) pour empêcher les remontées d'humidité au niveau de la ceinture (4).

Selon l'invention, les dits moyens (15, 16) pour empêcher les remontées d'humidité sont constitués par une barrière étanche, au moins aux liquides, formant un obstacle total et continu aux remontées de liquide, disposée transversalement au niveau des dites parties avant (9) et arrière (10), sur une largeur au moins égale à la largeur du matelas absorbant (7).

FIG.1a   FIG.1b

EP 0 352 207 A1

**Description**

L'invention est relative à une couche-culotte qui trouvera notamment son application dans le domaine de l'hygiène infantile et/ou médicale.

Dans ce domaine, l'aspect pratique et l'absence d'entretien procurés par les couches jetables ont largement contribué au développement et aux perfectionnements de celles-ci.

A cet égard, de telles couches culottes sont généralement formées à partir d'une bande complexe de matériau formée au moins par une feuille extérieure imperméable, une feuille intérieure perméable de confort et un matelas de matériau absorbant disposé entre les deux dites feuilles.

D'une manière courante, on utilise, pour former une telle bande, une feuille de polyéthylène et un voile de non-tissé entre lesquels est donc disposé le matériau absorbant à base de ouate de cellulose. Par ailleurs, de récents perfectionnements ont permis d'augmenter le coefficient de rétention de liquide des dits matelas absorbants. Ceci est rendu possible, d'une part, par une meilleure sélectivité de l'absorption au niveau de la ouate de cellulose et, d'autre part, par l'apport de produits superabsorbants.

Néanmoins, cette augmentation du pouvoir absorbant a mis en avant d'autres inconvénients, tels que la réjection des liquides vers l'extérieur de la couche, grévant ainsi le confort du porteur.

Pour pallier ces inconvénients, d'autres perfectionnements des couches culottes ont dû être mis en pratique et on connait des dispositifs "anti-fuites" au niveau de l'entrejambe et/ou au niveau de la ceinture de la couche.

Plus précisément, on a tout d'abord renforcé l'étanchéité au niveau de l'entrejambe du porteur en prévoyant différents systèmes élastiques d'entrejambes qui permettent d'adapter la forme de la couche à la morphologie du porteur.

Par ailleurs, au niveau de la ceinture, une première amélioration a été remarquée grâce à des dispositifs de fermeture adhésifs qui permettent de former une ceinture adaptable au mieux par rapport aux mensurations du porteur.

Grâce à ces dispositifs, on évite notamment l'entrebaillement de la couche qui, selon la position du porteur, peut être néfaste, en permettant des fuites lors d'un apport important instantané en liquide du fait de l'inertie du matériau absorbant.

Une autre amélioration au niveau de l'étanchéité de la ceinture de la dite couche a vu le jour grâce à la présence de moyens pour empêcher les remontées d'humidité placés de part et d'autre du matelas de matériau absorbant au niveau de la dite ceinture.

A ce sujet, il est connu des couches comportant une feuille de polymère imperméable, rapportée à chaque extrémité du matelas de matériau absorbant, entre le voile de non-tissé de la feuille intérieure perméable et la feuille extérieure imperméable, ce en recouvrant une partie du matelas absorbant côté porteur.

Plus précisément, ce film est maintenu en place par divers moyens de fixation ponctuelle, tels que collage, liage par embossage, soudures par ultrason, transversalement au niveau de la partie avant et de la partie arrière de la couche, entre la dite feuille perméable intérieure de non-tissé, et d'une part, le dit matelas de matériau absorbant, et/ou d'autre part, la dite feuille externe imperméable de polymère.

Toutefois, dans de telles réalisations, certains inconvénients subsistent au niveau de l'étanchéité et on ne constate pas une parfaite efficacité des moyens utilisés.

En effet, le film de polymère rapporté n'est pas fixé d'une manière totalement continue sur les matériaux, ce qui provoque des passages préférentiels pour les écoulements, généralement appelés "cheminées" de passage de liquide.

Par exemple, lorsque le morceau de film de polymère imperméable rapporté transversalement de part et d'autre du tampon absorbant est fixé par des lignes de "hot melt" parallèles, on forme ces "cheminées" entre le film de polymère et le dit film extérieur imperméable.

Toutefois, on remarque une certaine efficacité des moyens pour empêcher les remontées d'humidité du matelas de matériau absorbant, car le liquide ne sort pratiquement pas vers l'intérieur de la couche.

Cependant, à cause des dites "cheminées" créées entre les dits moyens et la feuille extérieure imperméable, rien n'empêche le liquide de sortir vers le haut de la couche lorsque le matelas a atteint un certain degré d'absorption, ce par exemple lors de mouvements du porteur qui comprime alors le matériau.

De plus, dans le cas des couches équipées d'élastiques aux entrejambes, étant donné leur processus de fabrication, à partir d'une bande en défilement continu, on est actuellement obligé de prévoir au moins deux "cheminées" au niveau de la ceinture.

En effet, la majorité des couches culottes, de nos jours, sont équipées d'élastiques longitudinaux au niveau de l'entrejambe pour éviter les fuites; or ces élastiques sont placés de part et d'autre du tampon absorbant, à l'état tendu, et sont liés sur une certain portion, sur le film extérieur, côté matelas absorbant.

Alors, on est en présence d'une bande complexe montrant des élastiques tendus tout au long de celle-ci dans le sens longitudinal de son défilement. Au moment de la découpe de la bande pour séparer une couche de celle-ci, ces élastiques, liés sur une certaine portion de la feuille extérieure se rétractent pour froncer la partie centrale de la couche au niveau de l'entrejambe.

Toutefois, il faut que les parties non liées des élastiques longitudinaux soient libres pour pouvoir se rétracter. C'est pourquoi, à ce jour, quelle que soit la structure des dits moyens pour empêcher les remontées d'humidité, on est obligé de prévoir des "cheminées", de part et d'autre du tampon élastique sensiblement dans l'axe de pose des élastiques, afin de les laisser se rétracter librement au moment de la découpe de la couche.

Ainsi, quelles que soient les réalisations connues à ce jour, les moyens pour empêcher les remontées d'humidité ne sont pas fiables à 100 % et ne procurent pas une étanchéité totale; des fuites et des remontées d'humidité apparaissant toujours au moins au niveau des dites "cheminées" d'élastiques.

Le but de la présente invention est de proposer une couche culotte, qui trouvera notament son application dans le domaine de l'hygiène infantile et/ou médicale, qui permette de pallier les inconvénients précités et qui autorise une augmentation du confort du porteur, en évitant les remontées d'humidité au niveau de la taille, en empêchant que le liquide ne sorte au niveau de la ceinture vers le haut et/ou vers l'intérieur de la couche.

En effet, selon la présente invention, la structure de la couche est telle qu'on forme un obstacle total et continu aux remontées de liquide au niveau de la ceinture avant et arrière, sur une largeur au moins égale à la largeur du matelas absorbant. Autrement dit, on supprime toutes les "cheminées" qui ont un effet néfaste vis-à-vis des réjections de liquide notamment lorsque le matériau absorbant atteint son degré de saturation et lorsque le porteur comprime le matelas intempestivement.

Un autre but de la présente invention est de proposer une couche culotte dont la structure permet toutefois sa fabrication selon des procédés en continu.

D'autres buts et avantages de la présente invention apparaitront au cours de la description qui va suivre qui n'est cependant donnée qu'à titre indicatif et qui n'a pas pour but de la limiter.

Selon la présente invention, la couche culotte, qui trouvera notamment son application dans le domaine de l'hygiène infantile et/ou médicale, formée d'une partie avant, et d'une partie arrière, réunies par une partie centrale constituant l'entrejambe, la dite couche présentant :

- une feuille extérieure imperméable, étanche au moins au liquide,
- une feuille intérieure perméable de confort,
- un matelas de matériau absorbant, disposé entre les deux dites feuilles,
- des moyens de fermeture, prévus au niveau des dites parties avant et/ou arrière, afin de constituer une ceinture pour la dite couche,
- des moyens élastiques longitudinaux, disposés de part et d'autre du dit matelas absorbant, au niveau de l'entrejambe,
- des moyens pour empêcher les remontées d'humidité, placés de part et d'autre du matelas de matériau absorbant, au niveau de la dite ceinture,
est caractérisée par le fait que les dits moyens pour empêcher les remontées d'humidité sont constitués par une barrière étanche, au moins au liquide, formant un obstacle total et continu aux remontées de liquide, disposée transversalement au niveau des dites partie avant et arrière, sur une largeur au moins égale à la largeur du matelas absorbant.

L'invention sera mieux comprise à la lectrue de la description suivante, accompagnée des figures qui en font partie intégrante.

Les figures 1a et 1b montrent schématiquement une couche culotte réalisée selon la présente invention et illustrent respectivement la face intérieure et la face extérieure de la couche.

La figure 2 représente une vue schématique partielle de la face extérieure, avec coupe partielle, d'une couche selon l'état de la technique connu.

La figure 3 représente une vue de dessus selon la flèche III-III de la figure 2.

La figure 4 montre une vue schématique partielle de la face intérieure d'une couche selon la présente invention, avec coupe partielle.

La figure 5 représente une vue en coupe selon l'axe V-V de la couche de la présente invention représentée à la figure 4.

La figure 6 représente une vue schématique selon la flèche VI-VI de la couche de la présente invention représentée aux figures 4 et 5.

La figure 7 montre une vue de dessus schématique illustrant le processus de fabrication des couches selon un premier mode de réalisation.

La figure 8 montre une coupe selon l'axe VIII-VIII de la figure 7 illustrant la sructure de l'élastique d'entrejambe.

La figure 9 montre une vue de dessus schématique illustrant le processus de fabrication des couches selon un deuxième mode de réalisation.

La figure 10 montre une coupe selon l'axe X-X de la figure 9, illustrant la structure des moyens élastiques d'entrejambe.

La figure 11 montre une vue de dessus schématique illustrant le processus de fabrication des couches selon un troisième mode de réalisation.

La figure 12 représente schématiquement une coupe longitudinale selon l'axe XII-XII de la figure 11 montrant en détail la pose de colle extensible sur film extérieur étanche plissé.

La présente invention vise une couche qui trouvera notamment son application dans le domaine de l'hygiène infantile et/ou médicale.

De nombreux perfectionnements ont été apportés à de telles couches culottes pour améliorer de plus en plus le confort du porteur.

Particulièrement, on a amélioré le pouvoir absorbant des liquides de la dite couche, et parallèlement, on a cherché à augmenter l'étanchéité des couches au niveau de l'entrejambe et de la ceinture pour éviter les réjections de liquide dans ces zones.

Une première solution pour éviter les fuites lors d'un apport instantané important de liquide a été trouvée en prévoyant des élastiques au niveau de l'entrejambe et de la ceinture pour maintenir le dit liquide dans la couche le temps que celui-ci soit absorbé par le matelas prévu à cet effet.

Par ailleurs, un deuxième phénomène se produit lorsque le matériau absorbant a quasiment atteint son degré d'absorption maximum, il s'agit des remontées d'humidité de liquide au niveau de la ceinture provoquées par exemple par des mouvements du porteur, qui alors compriment le matelas de matériau absorbant.

Il s'agit d'un phénomène voisin de celui de l'éponge; en effet, lorsque la quantité de liquide absorbé est importante, toute pression sur le matériau absorbant provoque une évacuation de liquide vers l'extérieur. De plus, étant donnée la structure de la couche, dont le matelas absorbant est prévu pour faciliter l'absorption longitudinal et autoriser un cheminement du liquide de la partie centrale vers les parties avant et/ou arrière, ces remontées d'humidité et de liquide ont tendance à s'échapper au niveau de la ceinture par l'intérieur et par le haut de la couche culotte.

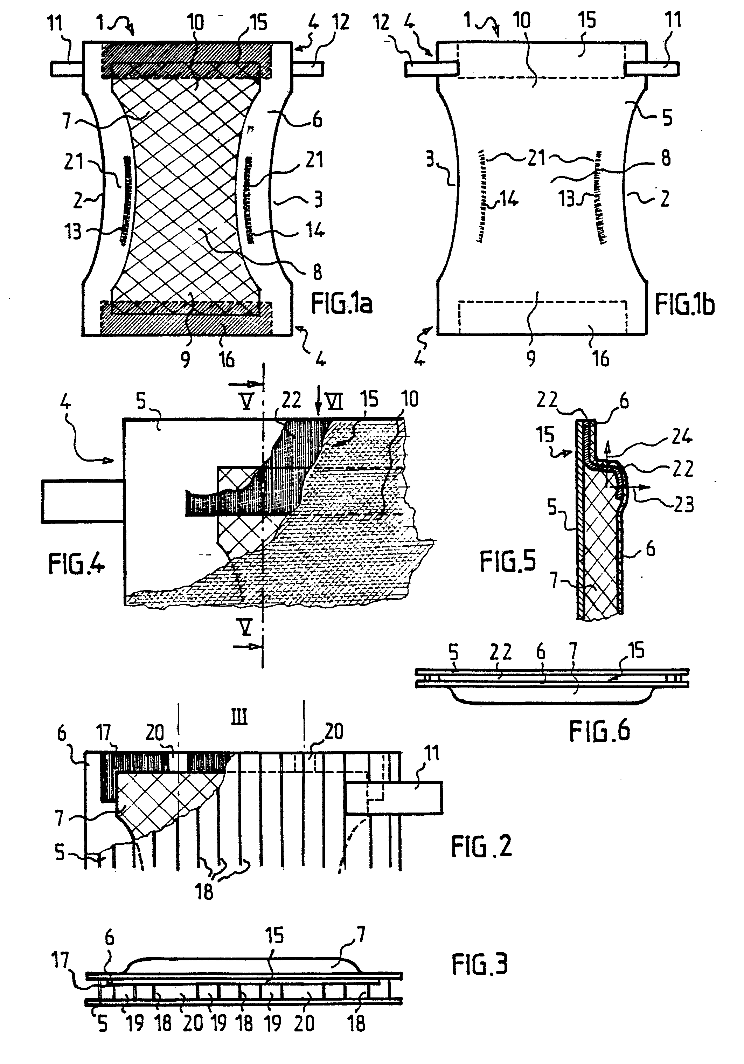

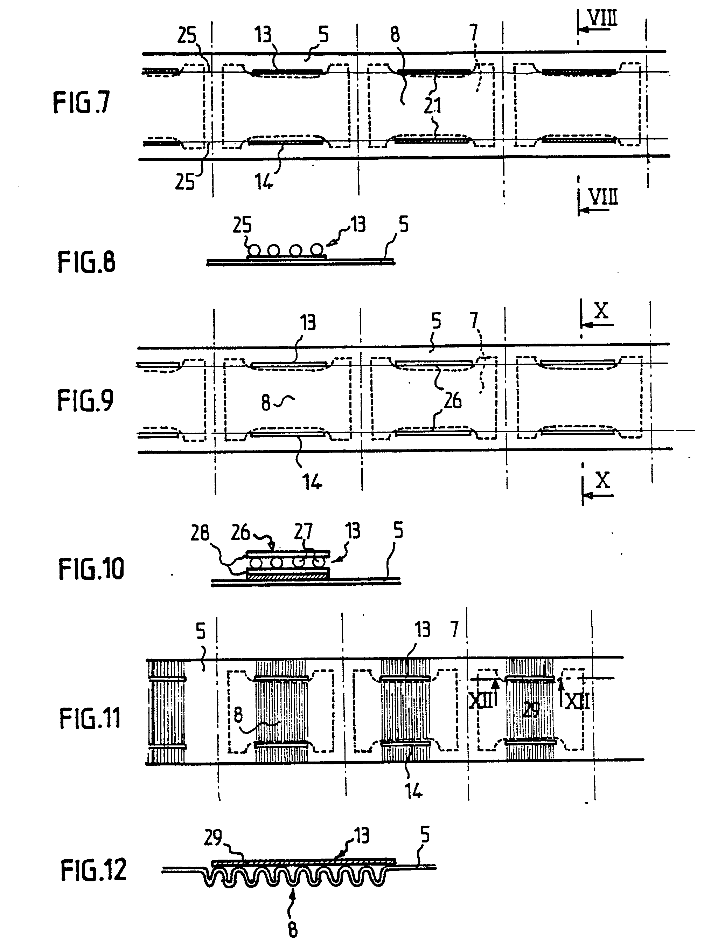

Pour remédier à cet inconvénient, certains fabricants ont développé une technique dans laquelle la couche culotte est équipée au niveau de la ceinture d'une feuille étanche rapportée en extrémité du matelas de matériau absorbant pour diminuer les réjections de liquide. Une telle réalisation est notamment illustrée aux figures 2 et 3 de la présente demande. Toutefois, certains inconvénients subsistent et on constate, dans certaines parties de la ceinture, des remontées d'humidité vers le haut et/ou vers l'intérieur malgré la présence de cette dite feuille étanche.

Les figures 1a et 1b illustrent une couche culotte 1 de type traditionnel, qui comporte des découpes latérales 2 et 3 pour permettre le passage des jambes, et qui peut être pliée telle que les extrémités 4 de la couche forment une ceinture apte à s'adapter à la morphologie du porteur.

En ce qui concerne la structure proprement dite de la couche, elle est formée traditionnellement par une feuille extérieure 5, imperméable, étanche au moins aux liquides, généralement réalisée par un film de polyéthylène, ainsi que par une feuille intérieure perméable de confort 6, destinée à s'appliquer vers l'utilisateur, généralement réalisée en voile de non-tissé. La figure 1a illustre la couche vue côté intérieur tandis que la figure 1b illustre la même couche vue de l'extérieur.

Par ailleurs, entre la feuille extérieure imperméable 5 et la feuille intérieure perméable 6, est disposé un matelas central de matériau absorbant 7, généralement constitué de ouate de cellulose ou tout autre matériau absorbant voire même hyperabsorbant. En outre, pour former une enveloppe dans laquelle est renfermé le dit matériau absorbant 7, les feuilles extérieure 5 et intérieure 6 sont fixées mutuellement sur la périphérie de la couche 1.

Ainsi formée, la couche culotte 1 montre une partie centrale 8, présentant les dites découpes 2 et 3 pour constituer l'entrejambe, réunissant d'une part une partie avant 9 et d'autre part une partie arrière 10.

De plus, pour constituer la ceinture 4, la couche culotte 1 présente des moyens de fermeture 11, 12, notamment prévus an niveau des parties avant 9 et/ou arrière 10. Par exemple, comme le montrent les figures, ces moyens de fermeture sont avantageusement constitué par des dispositifs adhésifs fixés sur la partie arrière de la couche 10. De tels moyens sont largement connus de l'Homme de l'Art.

Pour éviter les fuites au niveau de l'entrejambe 2, 3, la couche présente de préférence des moyens élastiques longitudinaux 13, 14 disposés de part et d'autre du dit matelas de matériau absorbant 7 au niveau de la partie centrale 8.

Enfin, pour ce qui est de l'étanchéité au niveau de la ceinture 4, la couche culotte 1 de la présente invention comporte des moyens pour empêcher les remontées d'huymidité disposés au moins de part et d'autre du matelas de matériau absorbant 7 au niveau de la ceinture 4.

Ces moyens sont représentés d'une manière générale sur les figures 1a et 1b. Par contre, les figures 2 et 3 illustrent la réalisation structurelle de tels moyens connus; tandis que les figures 4, 5, 6 montrent la réalisation de la présente invention.

Selon l'état de la technique connu, les moyens pour empêcher les remontées d'humidité 15 ou 16 sont constitués par un morceau de film de polymère 17, rapporté entre la feuille perméable de confort 6, et d'une part la feuille imperméable extérieure 6, puis d'autre part, le matelas de matériau absorbant 7, de façon à ce qu'à chaque extrémité de la couche 1, le dit film de polymère 17 couvre au moins partiellement une partie du matelas absorbant 7 du côté du porteur.

Ce film de polymère 17 est maintenu en place entre les dits éléments par des moyens de solidarisation non continus tels que lignes de collage, liage par embossage, soudures par ultrason.

A ce sujet, sur les figures 2 et 3, on a figuré les lignes de solidarisation en 18. Cependant, étant donné le type de fixation non continu, on forme alors entre le film de polymère 17 rapporté et la feuille imperméable extérieure 5 une multitude de "cheminées" 19.

Ces "cheminées" vont constituer un chemin préférentiel pour les remontées de liquide et malgré une certaine efficacité des moyens utilisés pour éviter les remontées d'humidité vers l'intérieur de la couche, au niveau de la ceinture, on aura inévitablement des remontées de liquide vers le haut au-travers de ces "cheminées" 19.

De plus, quelle que soit la structure des moyens de solidarisation utilisés, selon les techniques connues à ce jour, il est nécessaire de prévoir au moins deux autres "cheminées" 20 du fait de la présence des moyens élastiques 13, 14.

En effet, généralement, les couches culottes sont obtenues à partir d'une bande complexe, fabriquée en continu à partir des éléments de base, à savoir le film imperméable constituant la feuille extérieure 5, le voile de non-tissé constituant la feuille intérieure 6, et le matelas de matériau absorbant 7.

Les dits moyens élastiques longitudinaux 13, 14 sont alors placés de part et d'autre du matelas 7, à l'état tendu, et sont collés sur une certaine portion 21 au niveau l'entrejambe 2 ou 3.

Entre ces portions, les élastiques ne sont pas collés, mais restent tendus sur la bande complexe pendant tout son trajet. A l'issue du cycle de fabrication, les couches culottes 1 sont obtenues par sectionnement de la bande, ce qui provoque automatiquement le sectionnement des élastiques. A ce moment, il est donc nécessaire que les parties d'élastiques non collées puissent se rétracter afin d'obtenir les fronces souhaitées seulement au niveau de l'entrejambe.

C'est pourquoi, dans les couches connues telles que notamment représentées aux figures 2 et 3, on prévoit des zones de passage 20 des dits moyens élastiques longitudinaux 13, 14, constituant les dites "cheminées" pour autoriser leur rétraction après la découpe des élastiques au moment de la découpe de la dite bande en continu.

Ainsi, les moyens 15 ou 16 pour empêcher les remontées d'humidité sont inévitablement percés de deux "cheminées" qui constitueront des chemins préférentiels pour les remontées de liquide.

La couche culotte 1 de la présente invention pallie ces inconvénients et les dits moyens 15 ou 16 pour empêcher les remontées d'humidité sont constitués, au contraire, par une barrière 22 étanche au moins au liquide, formant un obstacle total et continu aux remontées de liquide, disposée transversalement au niveau des dites parties avant 9 et arrière 10, sur une largeur au moins égale à la largeur du matelas absorbant 7. Une telle réalisation est montrée en détail aux figures 4 à 6.

En effet, on retrouve une couche, réalisée d'une manière traditionnelle, présentant notamment une feuille extérieure imperméable 5, une feuille intérieure perméable 6, un matelas de matériau absorbant 7, des moyens de fermeture 11, 12 pour constituer la ceinture 4, des élastiques longitudinaux 13, 14 disposés au niveau de l'entrejambe 2, 3, et les dits moyens 15, 16 pour empêcher les remontées d'humidité au niveau de la ceinture 4.

Cependant, selon la présente invention, on ne laisse subsister aucune "cheminée" 19 ou 20 comme dans l'état de la technique connu. Au contraire, la barrière 22, selon la présente invention, réalise un scellage étanche continu entre les feuilles extérieure 5, intérieure 6 et le haut du matelas absorbant 7. Ceci est particulièrement illustré aux figures 5 et 6. De ce fait, on interdira toute remontée de liquide vers l'intérieur, comme simulé par la flèche 23, et/ou vers le haut, comme simulé par la flèche 24 sur la figure 5.

Dans un premier mode de réalisation de la présente invention, la dite barrière étanche 22 se présente sous la forme d'un fim de colle, étanche au moins aux liquides, déposé entre la feuille perméable intérieure 6 et, d'une part, le dit matelas de matériau absorbant 7, puis d'autre part, la dite feuille externe imperméable 5. De plus, comme le montrent particulièrement les figures 5 et 6, l'enduction de colle 22 est répartie et continue sur les trois matériaux considérés 5, 6, 7.

Il est à noter qu'on a obtenu de bons résultats en déposant une enduction de colle polymère sur la feuille intérieure perméable 6 avant qu'elle ne soit associée au matelas de matériau absorbant au cours du processus de fabrication. Ces dépôts sont synchronisés avec le défilement de la bande complexe pour s'appliquer sur les extrémités des couches avec un recouvrement du matelas absorbant.

A titre d'exemple, le dit film de colle 22 se présentera avantageusement sous la forme d'un adhésif, tel que notamment un polymère, de type "hot melt", sensible à la pression, filmogène, et à haute cohésion.

Ainsi, de tels dépôts de colle se lient très bien à la feuille imperméable extérieure et fournissent un scellage total et continu étanche des extrémités de la couche.

Dans un autre mode de réalisation, la dite barrière étanche 22 se présentera sous la forme d'un film de colle, étanche au moins aux liquides, élastique , déposé de la même manière que dans le premier mode de réalisation, à savoir entre la feuille perméable intérieure 6 et d'une part le dit matelas de matériau absorbant, puis d'autre part la feuille externe imperméable.

De même, le film de colle étanche élastique sera disposé d'une manière continue sur les trois matériaux considérés pour constituer la barrière étanche totale, sans cheminée.

Dans ce cas, on rendra avantageusement la ceinture de la couche élastique.

Le film de colle se présentera alors sous la forme d'un matériau polymère élastomérique et auto-adhésif de type "hot melt" obtenu par extrusion ou limite d'extrusion.

Il est à noter qu'une telle matière est élastique lorsqu'elle est sous tension c'est pourquoi la dite bande sera soit placée à l'état tendue, notamment sur la feuille externe imperméable non plissée, soit placée dans un état non tendu sur notamment la feuille externe imperméable qui sera alors plissée au moins partiellement.

Dans un autre mode de réalisation, on pourrait aussi envisager de réaliser la dite barrière étanche 22 sous la forme d'un film plastique étanche au moins aux liquides, disposé de manière identique à ce qui a été décrit précédemment, et maintenu tendu sur les trois matériaux 5, 6, 7 5 considérés par des moyens adhésifs continus adaptés.

Un tel film plastique se présentera avantageusement sous la forme d'un film polymère, tel que le polyéthylène, encollé de part et d'autre sur toute sa surface par un adhésif tel que décrit ci-dessus. Toutefois, l'épaisseur de l'enduction pourra être plus faible car l'étanchéité n'est plus réalisée seulement par le film de colle mais par le dit film plastique encollé.

Pour ce qui est des élastiques longitudinaux 13, 14, différentes solutions peuvent être adoptées, telles illustrées aux figures 7 à 12 de la présente demande.

Les figures 7 et 8 montrent une première solution que l'on peut considérer comme traditionnelle. En effet, les dits élastiques longitudinaux 13, 14 se présentent sous la forme d'au moins un brin élastique 25, collé à l'état tendu, de part et d'autre de la partie centrale 8 de la couche, sur les dites portions limitées 21.

Plus précisément, le ou les dits brins élastiques 25 sont collés, par un dépôt d'adhésif du type "hot melt" adapté au matériau, sur la feuille imperméable extérieure 5, ou sur le non-tissé 6, ce sur la face sur laquelle sera déposé ultérieurement le matelas absorbant 7. A ce sujet, les figures illustrent le matelas 7 en pointillé. Par contre, entre deux couches successives de la bande complexe, les élastiques sont maintenus tendus mais non collés.

A ce stade de la fabrication, après le collage, on coupera l'élastique en séparant légèrement la feuille imperméable 5 et l'élastique 25 afin de ne pas détériorer la dite feuille. Pour ce, on utilisera tout dispositif de sectionnement adapté permettant d'obtenir une coupe franche des élastiques.

Après découpe, les élastiques auront la capacité de pouvoir se rétracter; toutefois, la bande complexe en défilement étant maintenue tendue, ceci ne portera pas préjudice à la suite des opérations du processus de fabrication des couches.

Par ailleurs, selon cette technique des élastiques posés à l'état étiré d'un façon continue sur la feuille imperméable, on pourra également envisager de placer les élastiques sur la feuille imperméable non plus côté intérieur de la couche mais côté extérieur de la couche. Dans ce cas, il est à noter que les élastiques pourront être découpés à la fin de la fabrication, c'est-à-dire au moment de la séparation de la couche de la bande complexe;

Une autre mode de réalisation est illustré aux figures 9 et 10. Dans ce cas, les dits élastiques longitudinaux 13, 14 se présentent sous la forme d'une portion de ruban complexe thermo-rétractile 26 disposé de part et d'autre de la partie centrale 8 de la couche au niveau de l'entrejambe 2, 3.

A titre d'exemple, ce ruban complexe thermo-rétractile 26 sera avantageusement constitué de brins élastiques 27 étirés et fixés entre deux films de polymère 28, comme le montre précisément la figure 10.

A ce sujet, les brins élastiques sont solidarisés sur les dits films de polymère par exemple par soudures aptes à être détruites par apport d'énergie. Ainsi, le ruban complexe peut redevenir élastique par apport de chaleur afin de provoquer la rétraction et permettre la fonction souhaitée du fait de la désolidarisation brins 27-films 28.

En outre, ces portions de ruban thermo-rétractile sont disposées séquentiellement d'une façon synchrone au défilement de la bande et fixées notamment par un apport de colle, par exemple du type "hot melt".

Un troisième type de réalisation des élastiques longitudinaux 13, 14 est illustré aux figures 11 et 12.

Dans ce cas, les dits élastiques longitudinaux se présentent sous la forme d'un cordon de colle 29 élastique extensible, disposé préalablement sur la feuille imperméable extérieure 5, à l'état plissé, de part et d'autre de la partie centrale 8 de la couche.

En effet, il est connu un tel type de colle élastique de type "hot melt" apte à s'allonger mais qui ne peut pas se rétracter. Ainsi, en refroidissant, on retrouve une élasticité; cependant, il est alors nécessaire de disposer le cordon de colle sur une partie non plane pour permettre ultérieurement l'allongement du cordon élastique 29.

La figure 12 illustre le plissage transversal de la feuille imperméable 5 préalablement au dépôt de la colle 29. Après séchage de la colle, la feuille extérieure 5 pourra alors être retendue et on aura l'effet d'élasticité souhaité.

Naturellement, d'autres mises en oeuvre de la présente invention, à la portée de l'Homme de l'Art, auraient pu être envisagées sans pour autant sortir du cadre de la présente invention.

**Revendications**

1. Couche culotte (1) qui trouvera notamment son application dans le domaine de l'hygiène infantile et/ou médicale, formée d'une partie avant (9) et d'une partie arrière (10), réunies par une partie centrale (8) constituant l'entrejambe (2, 3), la dite couche présentant :
- une feuille extérieure imperméable (5), étanche au moins aux liquides,
- une feuille intérieure perméable de confort (6),
- un matelas de matériau absorbant (7), disposé entre les deux dites feuilles (5 et 6),
- des moyens de fermeture (11, 12) prévus au niveau des dites parties avant et/ou arrière afin de constituer une ceinture (4) pour la couche,
- des moyens élastiques (13, 14) longitudinaux, disposés de part et d'autre du dit matelas absorbant (7) au niveau de l'entrejambe (2, 3),
- des moyens (15, 16) pour empêcher les remontées d'humidité, placés de part et d'autre du matelas de matériau absorbant (7) au niveau de la dite ceinture (4),
caractérisée par le fait que les dits moyens (15, 16) pour empêcher les remontées d'humidité sont constitués par une barrière étanche (22), au moins au liquide, formant un obstacle total et continu aux remontées de liquide, disposée transversalement au niveau des dites parties avant (9) et arrière (10), sur une largeur au moins égale à la largeur du matelas absorbant (7).

2. Couche culotte selon la revendication 1, carctérisée par le fait que la dite barrière étanche (22) se présente sous la forme d'un film de colle étanche au moins aux liquides, déposé entre la dite feuille perméable intérieure (6) et, d'une part, le dit matelas de matériau absorbant (7), puis d'autre part, la dite feuille externe imperméable (5), l'enduction de colle (22) étant répartie et continue sur les trois matériaux considérés (5 à 7).

3. Couche culotte selon la revendication 2, caractérisée par le fait que le dit film de colle se présente sous la forme d'un adhésif "hot melt" sensible à la pression, filmogène, et à haute cohésion.

4. Couche culotte selon la revendication 1, caractérisée par le fait que la dite barrière étanche (22) se présente sous la forme d'un film de colle étanche au moins aux liquides, élastique, déposé entre la dite feuille perméable intérieure (6) et, d'une part, le dit matelas de matériau absorbant (7), puis d'autre part, la feuille externe imperméable 5, ledit film de colle étant disposé de façon continue sur les trois matériaux considérés (5 à 7).

5. Couche culotte selon la revendication 4, caractérisée par le fait que le dit film de colle étanche élastique se présente sous la forme d'un matériau polymère élastomérique et auto-adhésif de type "hot melt".

6. Couche culotte selon la revendication 1, caractérisée par le fait que la dite barrière étanche (22) se présente sous la forme d'un film plastique étanche au moins aux liquides, disposé entre la dite feuille perméable intérieure (6) et, d'une part, le dit

7. Couche culotte selon la revendication 6, caractérisée par le fait que le dit film plastique se présente sous la forme d'un film polymère tel que le polyéthylène.

8. Couche culotte selon la revendication 1, caractérisée par le fait que les dits élastiques longitudinaux (13, 14) se présentent sous la forme d'au moins un brin élastique (25), collé à l'état tendu, de part de d'autre de la partie centrale (8) de la couche (1).

9. Couche culotte selon la revendication 1, caractérisée par le fait que les dits élastiques longitudinaux (13, 14) se présentent sous la forme d'un cordon de colle élastique (29) extensible, disposé préalablement sur la feuille imperméable extérieure (5) à l'état plissé, de part et d'autre de la partie centrale (8) de la couche.

10. Couche culotte selon la revendication 1, caractérisée par le fait que les dits élastiques longitudinaux (13, 14) se présentent sous la forme de portion de ruban complexe (26) thermo-rétractile, constitué de brins élastiques (27) étirés et fixés entre deux films du polymère (28).

FIG.1a

FIG.1b

FIG.4

FIG.5

FIG.6

FIG.2

FIG.3

EP 0 352 207 A1

FIG.7

FIG.8

FIG.9

FIG.10

FIG.11

FIG.12

**Office européen des brevets**

# RAPPORT DE RECHERCHE EUROPEENNE

Numero de la demande

EP    89 44 0076

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | EP-A-059015 (THE PROCTER & GAMBLE COMPANY) * page 11, ligne 34 - page 13, ligne 5; figures 1-4 * | 1 | A61F13/15 |
| A | | 2-10 | |
| X | GB-A-2192832 (UNDERCOVER PRODUCTS INTERNATIONAL L.T.D.) * le document en entier * | 1 | |
| A | | 2-5 | |
| X | EP-A-252413 (KAO CORPORATION) * revendications 1-3; figures 1-4 * | 1 | |
| A | | 6, 7 | |
| X | EP-A-242766 (PAUL HARTMANN AG.) * abrégé; figure 1 * | 1 | |
| A | | 6, 7 | |
| A | EP-A-211197 (BOUSSAC SAINT FRERES B.S.F.) * revendications 1-11; figures 1-14 * | 1, 6-10 | |

### DOMAINES TECHNIQUES RECHERCHES (Int. Cl.5 )

A41B

Le présent rapport a été établi pour toutes les revendications

| Lieu de la recherche | Date d'achèvement de la recherche | Examinateur |
|---|---|---|
| LA HAYE | 17 OCTOBRE 1989 | KARIPIDOU C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
............................................
& : membre de la même famille, document correspondant

EPO FORM 1503 03.82 (P0402)